(19) **Europäisches Patentamt**
**European Patent Office**
**Office européen des brevets**

(11) **EP 3 017 752 B1**

(12) **FASCICULE DE BREVET EUROPEEN**

(45) Date de publication et mention
de la délivrance du brevet:
**02.11.2022 Bulletin 2022/44**

(21) Numéro de dépôt: **15190254.1**

(22) Date de dépôt: **16.10.2015**

(51) Classification Internationale des Brevets (IPC):
*A61B 5/00* (2006.01)    *A61B 5/087* (2006.01)
*A61N 1/36* (2006.01)    *A61B 5/08* (2006.01)
*A61H 23/00* (2006.01)    *A61N 1/365* (2006.01)

(52) Classification Coopérative des Brevets (CPC):
**A61B 5/4836; A61B 5/087; A61B 5/4818;**
**A61N 1/3611;** A61B 5/0809; A61H 23/00;
A61H 2230/405; A61N 1/36521

(54) **DISPOSITIF MÉDICAL ACTIF POUR LE TRAITEMENT SÉLECTIF ET PRÉCOCE DES HYPOPNÉES**

AKTIVE MEDIZINISCHE VORRICHTUNG FÜR DIE SELEKTIVE UND FRÜHZEITIGE BEHANDLUNG VON HYPOPNOEN

ACTIVE MEDICAL DEVICE FOR SELECTIVE, EARLY TREATMENT OF HYPOPNOEA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.10.2014 FR 1460320**

(43) Date de publication de la demande:
**11.05.2016 Bulletin 2016/19**

(73) Titulaires:
• **Sorin CRM SAS**
**92140 Clamart Cedex (FR)**
• **INSTITUT NATIONAL DE LA SANTE ET DE LA RECHERCHE MEDICALE (INSERM)**
**75013 Paris (FR)**
• **Université de Rennes 1**
**35065 Rennes Cedex (FR)**
• **Université Grenoble Alpes**
**38400 Saint-Martin-d'Hères (FR)**

(72) Inventeurs:
• **AMBLARD, Amel**
**92330 Sceaux (FR)**
• **GRAINDORGE, Laurence**
**44470 Thouaré sur Loire (FR)**
• **FEUERSTEIN, Delphine**
**92130 Issy les Moulineaux (FR)**
• **HERNANDEZ, Alfredo**
**35510 Cesson Sévigné (FR)**
• **PEPIN, Jean-Louis**
**38000 Grenoble (FR)**

(74) Mandataire: **Ungerer, Olaf**
**Page White & Farrer Germany LLP**
**Widenmayerstraße 10**
**80538 München (DE)**

(56) Documents cités:
WO-A2-2004/032719    US-A1- 2010 307 500
US-A1- 2012 253 249    US-A1- 2013 174 847
US-B1- 7 942 824

**Description**

L'invention concerne le diagnostic et le traitement des troubles respiratoires du sommeil, plus particulièrement ceux liés à une pathologie connue sous le nom de "syndrome d'apnée du sommeil" (SAS).

Le syndrome d'apnée du sommeil (SAS), ou plus précisément syndrome d'apnées-hypopnées du sommeil (SAHS) se manifeste par des arrêts (apnées) et/ou des diminutions (hypopnées) fréquentes - au moins 10 à 20 fois par heure - du flux respiratoire au cours de la nuit. Les hypopnées, tout comme les apnées, ont des effets sévères sur l'équilibre physiologique du patient, tels que risque d'hypoxémie, micro-éveil, etc.

Il convient de distinguer les deux troubles caractéristiques de cette pathologie, à savoir :

- les *apnées* (ou pauses respiratoires), définies comme étant des arrêts temporaires de la fonction respiratoire de durée supérieure à 10 s, survenant pendant une phase de sommeil du patient (car une apnée en état d'éveil ne peut pas être à l'origine d'un SAS) ; et
- les *hypopnées*, définies comme des décroissances importantes - mais sans interruption - du débit respiratoire pendant une phase de sommeil du patient.

La présente invention concerne spécifiquement le diagnostic et le traitement des hypopnées.

Une hypopnée est essentiellement caractérisée par :

i) une réduction significative de l'amplitude du débit respiratoire pendant une durée d'au moins 10 s, et
ii) une désaturation consécutive en oxygène d'au moins 3 à 4 % et/ou un micro-éveil consécutif.

Plus précisément, le seuil de réduction du débit respiratoire est en général fixé à 30 % pour une désaturation consécutive de 4 %, ou à 50 % de l'amplitude respiratoire de base pour une désaturation consécutive de 3 % (AASM Manual for The Scoring of Sleep and Associated Events, 2007). On notera que ces valeurs de seuil ne sont que des valeurs typiques, et peuvent varier d'un patient à l'autre, et pour un même patient évoluer au cours du temps, que ce soit au cours d'une même nuit ou d'une nuit à la suivante.

Dans la présente description, on utilisera le terme d'"'hypopnée" uniquement pour qualifier une réduction du débit ventilatoire suivie d'une désaturation en oxygène ou d'un micro-éveil (définition officielle d'une hypopnée selon l'American Academy of Sleep Medicine, publiée dans le Journal of Clinical Sleep Medicine, volume 8, n° 5, 2012).

Lorsque l'hypopnée n'est pas avérée mais seulement suspectée, du fait d'une détection précoce uniquement sur un critère de réduction du débit respiratoire, on utilisera alors les termes de "chute ventilatoire", ou encore "chute de l'amplitude respiratoire", "réduction (du débit) ventilatoire".

Les hypopnées constituent le trouble respiratoire le plus courant chez les patients sujets au SAS. Ainsi, un patient atteint d'un SAS sévère peut faire au cours d'une seule nuit une seule apnée mais jusqu'à 450 hypopnées. On connait diverses techniques de traitement des hypopnées, par des stimulations diverses qui ouvrent les voies respiratoires (en cas de troubles respiratoires obstructifs) ou stimulent les centres nerveux qui contrôlent la respiration (en cas de troubles respiratoires centraux).

Pour ce faire, il est essentiel de pouvoir détecter l'hypopnée automatiquement et en temps réel dès son apparition, avec la difficulté que la détection d'une chute ventilatoire n'est pas suffisante pour avérer l'hypopnée : en effet, on ne sera sûr que l'évènement était effectivement une hypopnée que quand cet événement sera achevé (en établissant alors qu'il vérifie bien les critères de durée, de désaturation en oxygène ou de survenue d'un micro-éveil).

Or, si la détection en temps réel des apnées est relativement facile, celle des hypopnées est plus difficile. En effet, les critères de définition d'une hypopnée incluent la désaturation en oxygène et le micro-éveil, qui n'ont lieu tous deux qu'après l'hypopnée donc, en tout état de cause, très longtemps après la détection de la chute respiratoire (typiquement, entre 10 et 60 secondes après), c'est-à-dire lorsque la respiration redevient normale. Il est donc trop tard pour traiter l'hypopnée puisque celle-ci a pris fin.

Il est certes envisageable de traiter de façon préventive, à bref délai, tous les événements de chute ventilatoire, par exemple 10 s après leur apparition, sans attendre la confirmation qu'il s'agit effectivement d'une hypopnée (qui implique la détection de la désaturation ou du micro-éveil). Cependant, tous les événements de chute ventilatoire ne sont pas nécessairement des hypopnées et, concrètement, les détecteurs en temps réel actuels ont une valeur prédictive positive qui ne dépasse pas 60 à 70 %. Autrement dit, pour un patient atteint d'un SAS sévère pouvant faire 400 hypopnées par nuit, un tel détecteur verra 570 événements et le patient sera donc traité inutilement 170 fois dans la nuit. Ce traitement excessif présente le double risque de déstructurer le sommeil du patient et de promouvoir l'effet d'accoutumance, les thérapies perdant de leur efficacité avec le temps. Ces effets et conséquences invalideraient les effets bénéfiques du traitement, et pourraient conduire à terme à une faible observance.

Pour éviter ce risque, il a été proposé de ne traiter que certains évènements, ou d'adapter le type de thérapie au type d'apnée ou hypopnée détectée. Ainsi :

- le EP 1 336 422 A1 (ELA Médical) prévoit de détecter une apnée ou une hypopnée à partir d'un signal ventilatoire obtenu par un stimulateur implanté. Si certaines conditions hémodynamiques cardiaques dépassent un seuil de référence, alors un ou plusieurs paramètres de stimulation cardiaque sont modifiés, tels que fréquence de stimulation, délai atrioventriculaire, etc. ;

- le US 2003/30153956 A1 prévoit également de détecter une apnée ou une hypopnée à partir d'un signal ventilatoire recueilli par un stimulateur implanté. Pour stopper le trouble respiratoire, la fréquence de base est ajustée, mais cet ajustement n'intervient qu'après détection d'un nombre minimal de troubles respiratoires avérés ;

- le US 7 371 220 B1 propose de détecter un événement d'apnée ou d'hypopnée par un dispositif implanté, en temps réel, dès l'apparition de la chute ventilatoire. Une thérapie différenciée est appliquée selon le type d'événement (d'origine obstructive ou d'origine centrale), mais il n'est en revanche pas prévu de sélection des événements à traiter, c'est-à-dire que la totalité des événements, qu'ils soient d'un type ou d'un autre, fait l'objet d'une thérapie.

Aucune de ces techniques ne s'est révélée pleinement satisfaisante pour améliorer le traitement des troubles respiratoires du sommeil, car ils ne ciblent pas les événements d'hypopnée justiciables d'une thérapie, et eux seuls. En particulier, aucune de ces techniques ne permet de traiter un maximum d'hypopnées en temps réel, sans risquer de compromettre l'efficacité de la thérapie par des thérapies abusives déclenchées par la détection de chutes ventilatoires qui ne correspondent pas, in fine, à des hypopnées avérées.

Les US 7 942 824 B1 et US 2010/307500 A1 visent plus spécifiquement la détection et le traitement des hypopnées, mais ils ne prévoient d'appliquer une thérapie qu'en cas d'hypopnée confirmée. Les techniques proposées par ces documents ne résolvent donc pas le problème évoqué plus haut, à savoir que la simple détection d'une chute ventilatoire (qui peut ne pas être annonciatrice d'une hypopnée) n'est pas suffisante en soi pour avérer la survenue d'une hypopnée, avec l'inconvénient que la thérapie n'est appliquée que de façon relativement tardive, c'est-à-dire après que l'hypopnée a complètement développé ses effets délétères.

Pour résoudre ce problème, l'invention propose une approche très différente de celles présentées jusqu'à présent, et propose d'opérer de façon automatique une analyse et une classification des chutes ventilatoires, détectées en temps réel, pour mieux cibler les hypopnées les plus sévères ou les plus certaines, et n'appliquer une thérapie qu'à ces dernières.

L'avantage d'une telle solution est non seulement d'éviter une surstimulation du patient, mais de pouvoir également, si une thérapie doit être appliquée, d'ajuster les paramètres de cette thérapie selon la typologie de l'hypopnée. Plus précisément, l'invention propose un dispositif médical actif selon la revendication 1.

Selon diverses caractéristiques subsidiaires avantageuses :

- le dispositif comprend en outre des moyens aptes à inhiber toute thérapie anti-hypopnée en cas d'attribution à l'événement courant d'un indicateur de non-suspicion a priori d'hypopnée ;

- le dispositif comprend en outre des moyens de mesure d'un signal physiologique fonction de l'activité respiratoire, des moyens de détection du franchissement d'un seuil limite par le signal physiologique, et des moyens pour forcer l'incrémentation du compteur à toute détection d'un événement de chute ventilatoire ;

- la période prédéterminée et/ou la valeur minimale prédéterminée sont des valeurs adaptatives variables, et le dispositif comprend en outre des moyens de calcul automatique de la période prédéterminée et/ou de la valeur minimale prédéterminée, respectivement, au cours de la nuit ;

- le dispositif comprend en outre des moyens de mesure d'un signal physiologique fonction de l'activité respiratoire, des moyens de détection du franchissement d'un seuil limite par un signal respiratoire ou par le signal physiologique, et des moyens pour forcer le déclenchement de la thérapie anti-hypopnée dès le franchissement dudit seuil limite ;

- le signal physiologique est un signal du groupe formé par: niveau de saturation sanguine en oxygène, taux de désaturation en oxygène sanguin, fréquence cardiaque, pression artérielle, onde de pouls, électroencéphalogramme, électromyogramme et les combinaisons des précédents ;

- les moyens discriminateurs comprennent des moyens aptes à analyser une pluralité de paramètres de l'événement de chute ventilatoire courant en fonction d'une pluralité de critères prédéfinis, des moyens aptes à attribuer, pour l'événement de chute ventilatoire courant, un score spécifique respectif à chacun des critères prédéfinis, des moyens aptes à établir un score composite global fonction d'une combinaison des scores spécifiques, et des moyens pour attribuer à l'événement courant un indicateur de suspicion a priori d'hypopnée ou bien de non-suspicion a priori d'hypopnée en fonction du résultat d'une comparaison du score global à un seuil de score prédéterminé ;

- les moyens discriminateurs comprennent en outre des moyens aptes à sélectionner en fonction de la valeur du score global une thérapie particulière parmi plusieurs thérapies possibles ;

- les au moins deux critères prédéfinis sont des critères du groupe formé par: historique des intervalles entre événements successifs de chute ventilatoire constitutifs d'épisodes d'hypopnées ; sévérité de l'événement de chute ventilatoire courant; origine physiologique, obstructive ou centrale, de l'évènement de chute ventilatoire; stade de sommeil courant du patient; et historique du degré d'efficacité des thérapies appliquées.

On va maintenant décrire un exemple de mise en oeuvre de la présente invention, en référence aux dessins annexés où les mêmes références désignent d'une figure à l'autre des éléments identiques ou fonctionnellement semblables.

La Figure 1 illustre schématiquement les différents éléments constitutifs d'un dispositif permettant la mise en oeuvre de l'invention.

Les Figures 2a et 2b sont des histogrammes de relevés cliniques détaillant, pour deux patients respectifs différents, la répartition des épisodes, c'est-à-dire des intervalles entre hypopnées successives au cours d'une nuit de sommeil.

La Figure 3 est un schéma illustrant un premier aspect de mise en oeuvre
de l'invention, par une technique reposant sur l'analyse de la succession dans le temps des événements d'hypopnée pour décider de déclencher, ou
non, une thérapie en cas de survenue d'une chute ventilatoire.

La Figure 4 est un schéma détaillant les étapes mises en oeuvre par la technique illustrée dans son principe Figure 3.

La Figure 5 est un schéma illustrant un autre aspect de mise en oeuvre de l'invention, par une technique reposant sur l'analyse du profil de la chute ventilatoire détéctée et sa comparaison avec un profil de référence pour décider de déclencher, ou non, une thérapie en cas de survenue d'une chute ventilatoire.

La Figure 6 est un schéma détaillant un autre exemple de mise en oeuvre de l'invention, basée sur une technique multicritère où la chute ventilatoire est analysée en temps réel et simultanément confrontée à une pluralité de critères, donnant des indices respectifs qui seront pondérés et combinés afin de décider de déclencher, ou non, une thérapie en cas de survenue d'une chute ventilatoire.

On va maintenant décrire un exemple de réalisation du dispositif de l'invention.

En ce qui concerne ses aspects logiciels, l'invention peut être mise en oeuvre par une programmation appropriée du logiciel de commande d'un dispositif médical actif, implanté ou externe, pourvu de fonctions de thérapie des troubles respiratoires du sommeil.

Ces dispositifs comprennent un microprocesseur programmable associé à des circuits d'analyse de données recueillies par dives capteurs et de délivrance de thérapies anti-apnée et anti-hypopnée. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé correspondant à l'opération du dispositif de l'invention est mis en oeuvre par des moyens principalement logiciels, au moyen d'algorithmes appropriés exécutés par un micro-contrôleur ou un processeur numérique de signal. Pour la clarté de l'exposé, les divers traitements appliqués seront décomposés et schématisés par un certain nombre de blocs fonctionnels distincts interconnectés, mais cette représentation n'a toutefois qu'un caractère illustratif, ces circuits comprenant des éléments communs et correspondant en pratique à une pluralité de fonctions globalement exécutées par un même logiciel.

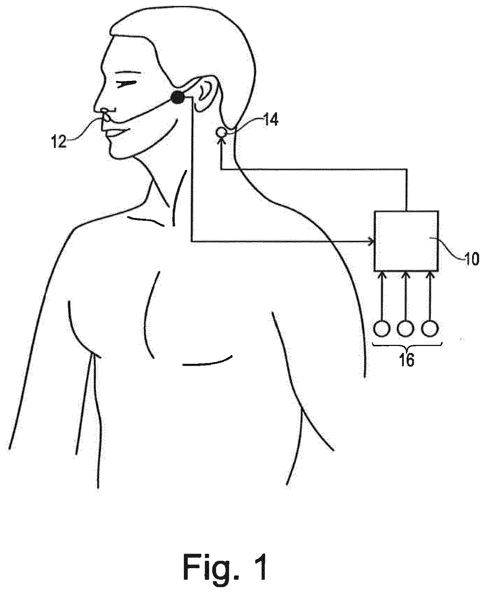

La Figure 1 illustre schématiquement les principaux éléments nécessaires à la mise en oeuvre de l'invention.

Cette mise en oeuvre est opérée par un dispositif 10, qui peut être un dispositif externe, par exemple un enregistreur Holter relié à divers capteurs ou électrodes (ou bien implanté, par exemple un stimulateur cardiaque, resynchroniseur et/ou défibrillateur).

Ce dispositif 10 est pourvu de moyens de mesure du débit respiratoire. Dans le cas d'un dispositif externe, il peut s'agir par exemple d'une canule de pression nasale 12 (et/ou d'une canule buccale) ou autre type de capteur tel que thermistance ou capteur mécanique des variations de volume de l'abdomen et/ou de la cage thoracique (au moyen d'une ceinture équipée de capteurs sensibles à l'étirement). Dans le cas d'un dispositif implanté, les moyens de mesure du débit respiratoire peuvent être, de façon conventionnelle, des moyens de mesure de l'impédance transthoracique entre le boitier de l'implant et une électrode distante.

Le dispositif 10 est en outre relié à un moyen d'application d'une thérapie anti-hypopnée. Il peut s'agir notamment - mais de façon aucunement limitative - d'un effecteur kinesthésique 14, constitué par exemple d'un vibreur placé dans une région sensible de l'épiderme, typiquement (chez l'adulte) dans la région de l'os mastoïde au voisinage de l'oreille. La stimulation vibrotactile appliquée sur la peau par l'effecteur 14 est détectée par les récepteurs sensoriels ou mécanorécepteurs de l'organisme, et transmise par l'intermédiaire des nerfs sensitifs au système nerveux central autonome.

Le dispositif 10 est également relié à divers capteurs 16 tels qu'électrodes, fibres optiques, accéléromètres, microphones, etc. permettant de mesurer des signaux physiologiques tels que la fréquence cardiaque, la saturation en oxygène (mesurée par un capteur placé sur le doigt ou sur l'oreille), le phonocardiogramme, l'électroencéphalogramme ou l'électromyogramme, afin de disposer, outre la mesure de débit respiratoire, de signaux additionnels qui permettront d'une part de mieux discriminer les chutes ventilatoires annonciatrices d'hypopnées, et d'autre part de déterminer *a posteriori* si l'évènement détecté à l'origine de la chute ventilatoire était ou non une hypopnée.

L'invention vise spécifiquement le diagnostic et le traitement des hypopnées (et non celui des apnées), en particulier suite à la détection précoce d'une chute ventilatoire, et ceci sans attendre la fin de l'évènement (donc sans savoir *a priori* s'il s'agit ou non d'une véritable hypopnée).

Il s'agit notamment de discriminer entre chute ventilatoire annonciatrice d'une véritable hypopnée - dans ce cas, justiciable d'une thérapie immédiate - ou non, auquel cas il conviendra de n'appliquer aucune thérapie pour éviter inutilement l'apparition d'effets indésirables tels que toux, micro-éveil, etc.

Les différentes thérapies susceptibles d'être appliquées à une hypopnée avérée sont en elles-mêmes connues, et ne font pas partie de la présente invention. L'invention a en effet pour objet de déclencher, ou non, l'application d'une thérapie à un stade avancé d'une hypopnée suspectée (la suspicion provenant de la détection d'une chute ventilatoire), éventuellement en opérant une sélection entre plusieurs thérapies possibles, mais sans modifier le mode d'application de cette thérapie.

Pour mémoire, dans le cas d'un dispositif externe, il peut s'agir d'une thérapie par stimulation kinesthésique, comme décrit par exemple dans le WO 2007/141345 A1 (FR 2 908 624 A1). Dans le cas d'un dispositif implanté, il peut s'agir d'une thérapie par modification de la fréquence de stimulation cardiaque et/ou du délai atrioventriculaire.

Selon l'invention, dès qu'une chute ventilatoire est détectée, on considère qu'il y a suspicion d'hypopnée (hypopnée qui ne pourra être avérée qu'après la fin de l'évènement, donc de façon très tardive) et l'on opère alors une analyse permettant de décider précocement, c'est-à-dire sans attendre la fin de l'évènement, du déclenchement ou non d'une thérapie anti-hypopnée et de s'abstenir de toute thérapie pour des événements où la chute ventilatoire n'est probablement pas annonciatrice d'une hypopnée.

Cette analyse, pour décider le déclenchement ou non d'une thérapie, peut être fondée sur plusieurs stratégies, alternatives ou cumulatives, correspondant à divers critères de diagnostic que l'on va décrire en détail ci-après.

*Critère n°1 : succession en épisodes des événements d'hypopnée*

Les diagnostics de polysomnographie révèlent que les événements d'hypopnée surviennent par "épisodes" c'est-à-dire en succession à peu près régulière à l'intérieur d'intervalles temporels relativement courts, les différents épisodes étant séparés par des pauses plus longues. Un premier critère consiste à évaluer si la chute ventilatoire détecté correspond à un tel "épisode", afin de n'appliquer une thérapie que dans ce cas, de façon à traiter un maximum d'hypopnées tout en limitant le risque de thérapies inutiles qui pourraient avoir des effets délétères.

Il convient donc de déterminer si la chute ventilatoire détectée est isolée dans le temps, ou bien si elle fait partie d'un "épisode".

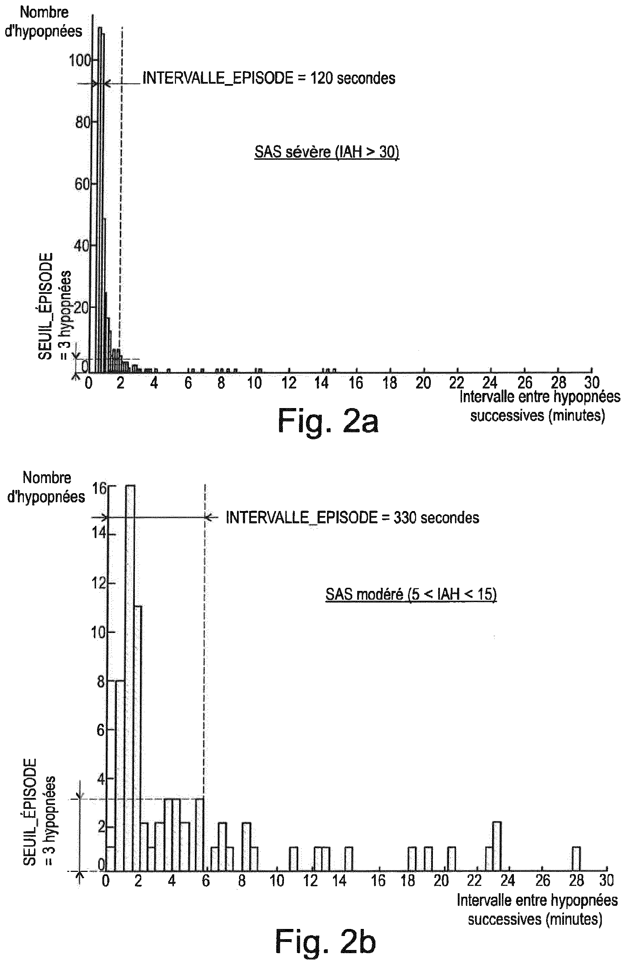

Le phénomène des épisodes présente toutefois une très grande variabilité d'un patient à l'autre, comme le montrent les Figures 2a et 2b qui sont des histogrammes de relevés cliniques détaillant, pour deux patients respectifs différents, la répartition des épisodes, c'est-à-dire des intervalles entre hypopnées successives au cours d'une nuit de sommeil.

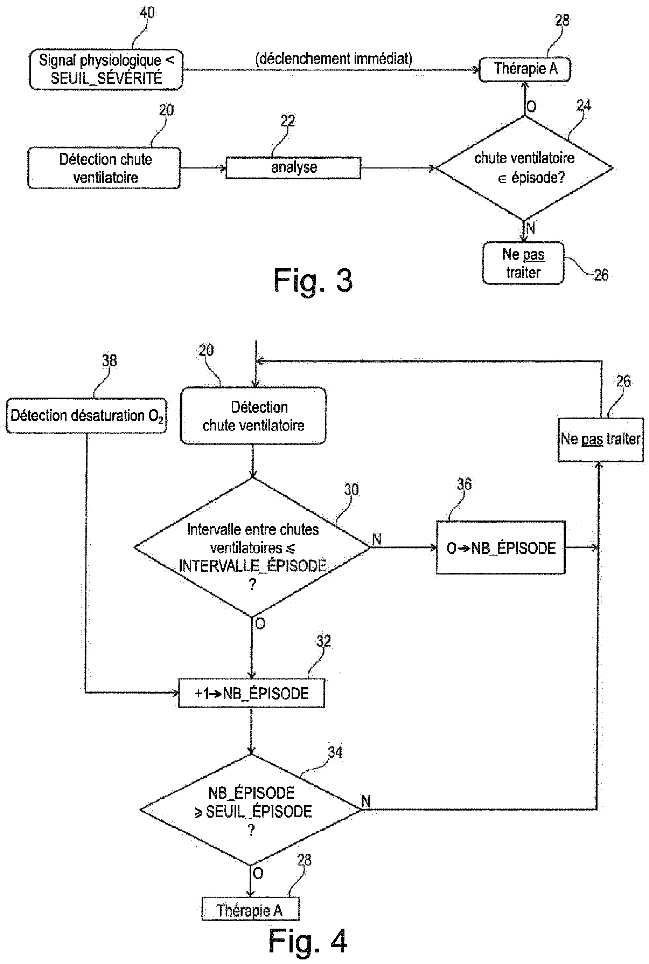

Le procédé d'analyse correspondant est illustré Figures 3 et 4.

Comme illustré Figure 3, sur détection d'une chute ventilatoire (bloc 20), cette chute ventilatoire est analysée (bloc 22) : si elle est considérée comme n'appartenant pas à un épisode (test 24), aucune thérapie n'est appliquée (bloc 26). Dans le cas contraire, une thérapie anti-hypopnée prédéterminée ("thérapie A") est appliquée (bloc 28).

Plus précisément, comme illustré Figure 4, sur détection d'une chute ventilatoire (bloc 20), l'algorithme évalue le temps écoulé depuis la dernière chute ventilatoire, et vérifie que l'intervalle de temps séparant ces deux chutes ventilatoires successives ne dépasse pas un intervalle de temps prédéterminé INTERVALLE_ÉPISODE (bloc 30). Si tel est bien le cas, la chute ventilatoire est considérée comme appartenant bien à un épisode d'hypopnées et un compteur NB_ÉPISODE du nombre d'hypopnées dans l'épisode est incrémenté (bloc 32). Dans le cas où une apnée (qui peut être considérée comme une forme extrême d'hypopnée) est détectée durant l'intervalle de temps INTERVALLE_ÉPISODE, alors le compteur NB_ÉPISODE est incrémenté également (tandis que l'apnée pourra être traitée en tant qu'apnée dès son apparition).

La valeur du compteur NB_ÉPISODE est ensuite comparée à un seuil SEUIL_ÉPISODE (bloc 34), correspondant à un seuil minimal de confiance et, si tel est bien le cas, la thérapie est déclenchée (bloc 28).

Si au test 30 on a constaté que l'intervalle séparant la chute ventilatoire que l'on vient de détecter de celle qui la précédait dépasse la valeur INTERVALLE_ÉPISODE, alors on remet à zéro le compteur d'hypopnées NB_ÉPISODE (bloc 36). La chute ventilatoire, isolée, ne fera pas l'objet d'une thérapie (bloc 26).

On notera que les valeurs INTERVALLE_ÉPISODE et SEUIL_ÉPISODE peuvent être adaptées au patient par un paramétrage faisant par exemple suite à une polysomnographie de diagnostic, préalable à la mise en place du système automatique. Ainsi, dans le cas d'un patient atteint d'un SAS sévère (indice d'apnée-hypopnée IAH > 30), INTERVALLE_ÉPISODE peut être fixé à 120 s, et SEUIL_ÉPISODE à 3 hypopnées. Pour un patient atteint d'un SAS modéré (5 < IAH < 15), INTERVALLE_ÉPISODE peut être fixé à 330 s, et SEUIL_ÉPISODE à 3 hypopnées.

On notera par ailleurs que ces deux paramètres peuvent évoluer pour un même patient qui, s'il est répondeur au traitement, pourra voir INTERVALLE_ÉPISODE et/ou SEUIL_ÉPISODE augmenter. Cette adaptation au cours du temps peut se faire lors d'une analyse, automatique ou manuelle, des résultats de la détection des hypopnées stockés en fin de nuit et consultables par un médecin. Il est également possible, si le médecin constate une trop grande discordance du nombre de chutes ventilatoires ayant déclenché une thérapie par rapport au nombre d'hypopnées avérées (c'est-à-dire avec désaturation et/ou micro-éveil en fin d'hypopnée) de réajuster ces paramètres.

Dans une variante de mise en oeuvre, les valeurs INTERVALLE_ÉPISODE et/ou SEUIL_ÉPISODE sont recalculées automatiquement au cours de la nuit. Par exemple, les propriétés statistiques des événements détectés et avérés peuvent être analysées. Les intervalles entre hypopnées consécutives suivant vraisemblablement une loi de Poisson (cf. Figure 2A) qui peut être paramétrée, ces paramètres peuvent être actualisés progressivement au cours de la nuit pour définir une valeur INTERVALLE-ÉPISODE adaptative à partir d'une formulation analytique de la loi statistique.

Si le patient est muni d'un capteur permettant de détecter une désaturation en oxygène (typiquement, une baisse d'au moins 3 % de la saturation en oxygène par rapport à la saturation de base), il est possible d'utiliser cette information, qui est un critère de confirmation d'hypopnée, pour améliorer le fonctionnement de l'algorithme d'analyse des épisodes d'hypopnées.

En effet, la détection de la désaturation, qui survient après l'hypopnée et donc entre deux hypopnées successives, confirme que la chute ventilatoire précédente était bien annonciatrice d'une hypopnée, ce qui renforce la nécessité d'une thérapie. En conséquence, notamment pour un patient atteint d'un SAS sévère, il est possible d'intégrer un critère supplémentaire à la détection d'un épisode, un épisode pouvant être défini par exemple comme constitué de trois hypopnées se répétant à moins de 120 s d'intervalle, ou bien comme deux hypopnées se répétant à moins de 120 s, s'il y a eu pendant ces 120 s détection d'une désaturation en oxygène. Pour cela, comme illustré Figure 4, la détection d'une désaturation en oxygène (bloc 38) force l'incrémentation du compteur NB_ÉPISODE (bloc 32), indépendamment de la détection d'une chute ventilatoire.

On notera que ce cas n'est pas limité à la désaturation en oxygène : par exemple si le capteur utilisé est du type EEG il peut s'agir d'un micro-éveil, et tout micro-éveil détecté entre deux hypopnées successives d'un même épisode incrémentera également le compteur NB_ÉPISODE.

*Critère n°2 : sévérité de l'évènement lié à la chute ventilatoire détectée*

Conformément à ce second critère, l'analyse exposée plus haut (qui a pour objet de déterminer si l'on se trouve ou non à l'intérieur d'un épisode d'hypopnée) est contournée de manière à provoquer le déclenchement immédiat et sans autre condition d'une thérapie si un signal physiologique passe en deçà d'un seuil de sévérité SEUIL_SÉVÉRITÉ prédéterminé (bloc 40, Figure 3), auquel cas la chute ventilatoire est immédiatement traitée (bloc 28), sans attendre la confirmation de l'analyse en épisodes. Par exemple, si l'on détecte une saturation sanguine en oxygène en deçà de 90 % de la saturation de base, cette mesure est un signe de pathologie sévère imposant une thérapie immédiate.

De même, en cas de réduction très significative du débit ventilatoire, voire une réduction dégénérant en apnée, alors l'évènement sera justiciable d'une thérapie immédiate.

Un autre exemple possible de signal physiologique est le rythme cardiaque : si celui-ci ralentit en deçà d'un seuil, alors une thérapie est immédiatement appliquée.

Dans tous les cas, le seuil de déclenchement est un paramètre déterminé initialement par le médecin, mais qui peut être modifié, automatiquement ou manuellement, en fonction de l'évolution de la maladie et de l'efficacité du traitement.

On notera par ailleurs que ce critère de sévérité a priorité sur les autres critères décrits ci-après, de sorte que si ce critère est vérifié, alors la thérapie sera déclenchée immédiatement dès détection de la chute ventilatoire.

*Critère n°3 : profil caractéristique de l'évènement*

On constate en général que les hypopnées se répètent au cours d'une même nuit de façon stéréotypée, avec une amplitude, une durée, une morphologie, etc. constituant un "profil" caractéristique d'un même patient. De ce fait, il est possible d'utiliser un critère basé sur l'analyse du profil de l'hypopnée, afin de ne déclencher une thérapie que si la chute ventilatoire correspond bien au profil caractéristique enregistré pour le patient, tandis que les nouveaux types de profils, non reconnus, ne feront pas l'objet d'une thérapie. Ces nouveaux profils seront en revanche enregistrés pour élargir le nombre de profils mémorisés, et seront ensuite considérés comme des profils connus qui, s'ils se répètent, pourront à leur tour constituer un critère de déclenchement d'une thérapie.

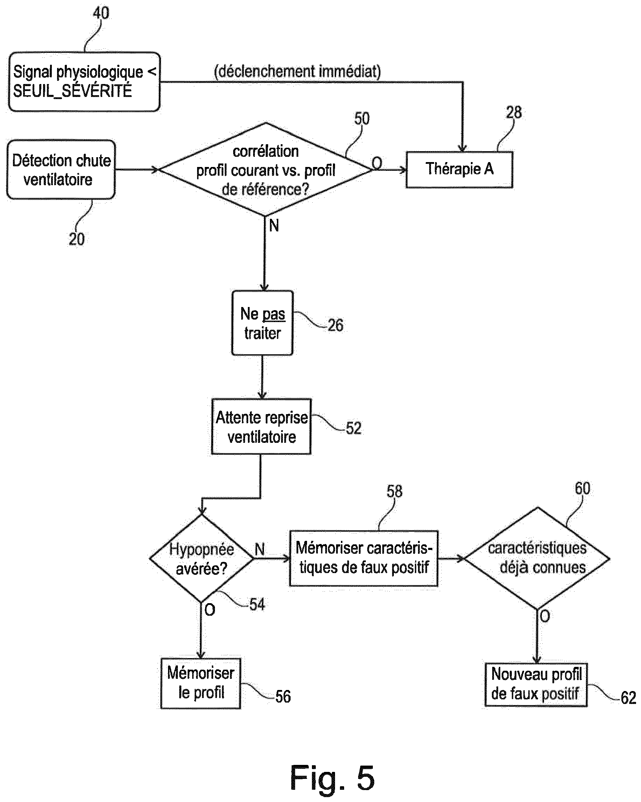

La Figure 5 illustre de façon plus détaillée la manière dont ce critère basé sur l'analyse de profil est mis en oeuvre.

Plusieurs aspects de profil d'hypopnées peuvent être identifiés chez un patient, fonction de :

- l'amplitude de la chute ventilatoire ;
- la morphologie des cycles respiratoires avec chute ventilatoire, ces cycles présentant une forme plus aplatie et carrée, tandis que les cycles normaux présentent une forme plus ronde ;
- la vitesse, soudaine ou bien progressive, de la chute ventilatoire, et le fait qu'elle soit ou non précédée d'une hyperpnée ;

- la durée de la chute ventilatoire ;
- la présence ou non d'hyperpnées entre hypopnées et entre apnées. Un certain nombre de profils correspondants à ces aspects sont conservés dans une mémoire du dispositif.

Dès qu'une chute ventilatoire est détectée (étape 20), le dispositif vérifie (test 50) si ses caractéristiques correspondent ou non à celles d'un des profils enregistrés, par exemple en calculant une fonction de corrélation entre le profil courant et le profil de référence et en vérifiant si le degré de corrélation dépasse ou non un seuil donné. Dans l'affirmative, une thérapie est appliquée (bloc 28).

Si une chute ventilatoire est détectée mais qu'elle ne correspond pas à l'un des profils enregistrés, aucune thérapie n'est appliquée (bloc 26). Le dispositif attend alors la fin de l'évènement (bloc 52) et détermine, à partir des critères de désaturation et/ou de micro-éveil, si cet événement était ou non une hypopnée (test 54) :

- dans l'affirmative (ce qui signifie que l'hypopnée n'a pas été suspectée en dépit de la chute ventilatoire détectée), alors un nouveau profil de référence est créé et stocké dans la mémoire du dispositif (bloc 56), de manière à améliorer l'efficacité du dispositif ;
- dans le cas contraire (ce qui signifie que la chute ventilatoire n'était pas annonciatrice d'une hypopnée), le profil est conservé en mémoire (bloc 58) et si ce type d'événement se reproduit (test 60), un profil de faux positif est créé et sauvegardé (bloc 62), ces informations pouvant être utilisées ultérieurement par le praticien pour reparamétrer le dispositif afin d'en améliorer la spécificité.

Dans cet exemple aussi, le critère de sévérité de l'événement peut être activé, en lui donnant une priorité supérieure au critère d'analyse du profil de manière que la détection du franchissement de seuil de sévérité (bloc 40) provoque le déclenchement immédiat de la thérapie (bloc 28).

*Critère n° 4 : origine physiologique de l'hypopnée*

De même que les apnées, les hypopnées peuvent être de deux origines :

- obstructive, lorsque l'hypopnée est due à une obstruction des voies aériennes, probablement causée par une faiblesse musculaire ;
- centrale, lorsque l'hypopnée est causée par un défaut de commande de la respiration au niveau du système nerveux central.

La classification entre hypopnées obstructives et hypopnées centrales peut être utilisée comme critère pour décider ou non d'appliquer une thérapie.

En effet, on constate chez certains patients une forte prédominance d'événements obstructifs, tandis que d'autres ont une forte prédominance d'événements de type central. De ce fait, la survenue d'une chute ventilatoire présentant les caractéristiques d'un événement non prédominant peut être due à une fausse détection, et on choisira dans ce cas de ne pas appliquer de thérapie : par exemple, si un patient est considéré comme faisant des hypopnées obstructives dans 90 % des cas, la détection d'une chute ventilatoire typique d'une hypopnée centrale ne déclenchera pas de thérapie.

La détermination de l'origine de l'hypopnée peut être opérée en temps réel par une analyse de profil de même nature que ce qui a été exposé ci-dessus pour le critère n°3, notamment sur la base des caractéristiques suivantes (on pourra notamment se référer à l'article de Renderath et al. paru dans Sleep, 2013 ; 36(3) : 363-368) :

- morphologie des cycles respiratoires : en cas d'hypopnée, une forme écrasée de la courbe d'inspiration révèle une origine obstructive ;
- respiration paradoxale : une hypopnée obstructive peut entrainer un déphasage entre les mouvements de respiration du thorax et ceux de l'abdomen ;
- reprise ventilatoire : graduelle pour une hypopnée centrale, abrupte pour une hypopnée obstructive ;
- séquencement des micro-éveils : ceux-ci apparaissent au début de la reprise ventilatoire dans le cas d'une hypopnée obstructive, et au maximum de la reprise ventilatoire dans le cas d'une hypopnée centrale ;
- stade de sommeil après le micro-éveil : le sommeil paradoxal est plus propice aux hypopnées obstructives.

La classification entre hypopnée obstructive et centrale peut servir par ailleurs de critère pour différencier la thérapie appliquée.

Ainsi, dans le cas d'une hypopnée centrale, il sera préférable de délivrer une thérapie activant le diaphragme, par exemple par stimulation du nerf phrénique, tandis que pour une hypopnée obstructive il sera préférable de stimuler les muscles des voies aériennes supérieures, par exemple via le nerf hypoglosse ou le nerf laryngé.

*Critère n° 5 : stade de sommeil*

L'état de veille ou de sommeil, ainsi que les différents stades de sommeil, affectent le rythme respiratoire.

En particulier, lors des périodes de veille ou d'endormissement, voire de sommeil paradoxal, la respiration n'est pas stable et l'on peut observer des variations soudaines et importantes, mais non pathologiques, des périodes et des amplitudes des cycles respiratoires.

Dès lors, les changements de rythme et d'amplitude respiratoires peuvent ne pas être liés à des hypopnées et il sera préférable dans ce cas de suspendre l'application d'une thérapie.

En revanche, les chutes ventilatoires survenant en sommeil lent pourront être traitées avec une priorité supérieure, la chute ventilatoire étant dans ce cas avec une forte probabilité annonciatrice d'une hypopnée.

*Critère n° 6 : efficacité du traitement*

Lorsqu'un certain nombre d'hypopnées suspectées (chutes ventilatoires détectées) ne répond pas au traitement anti-hypopnée au cours de la nuit (ou de nuits successives), cette typologie de réduction de débit doit être mémorisée pour analyse ultérieure. S'il s'avère que ce ne sont pas des hypopnées avérées *a posteriori*, alors il ne faut plus les détecter (faux positifs comme dans le cas du critère n° 3). Si par contre, il s'avère que ce sont des hypopnées avérées, alors une alerte peut être émise de manière à informer le praticien que les thérapies initialement appliquées ne sont peut-être plus appropriées. Dans tous les cas, le traitement de ce type de chutes du débit ventilatoire doit être suspendu.

*Utilisation combinée des critères n° 1 à 6*

Les critères qui ont été décrits plus haut peuvent être utilisés conjointement pour prendre la décision d'appliquer ou non une thérapie sur détection d'une chute ventilatoire.

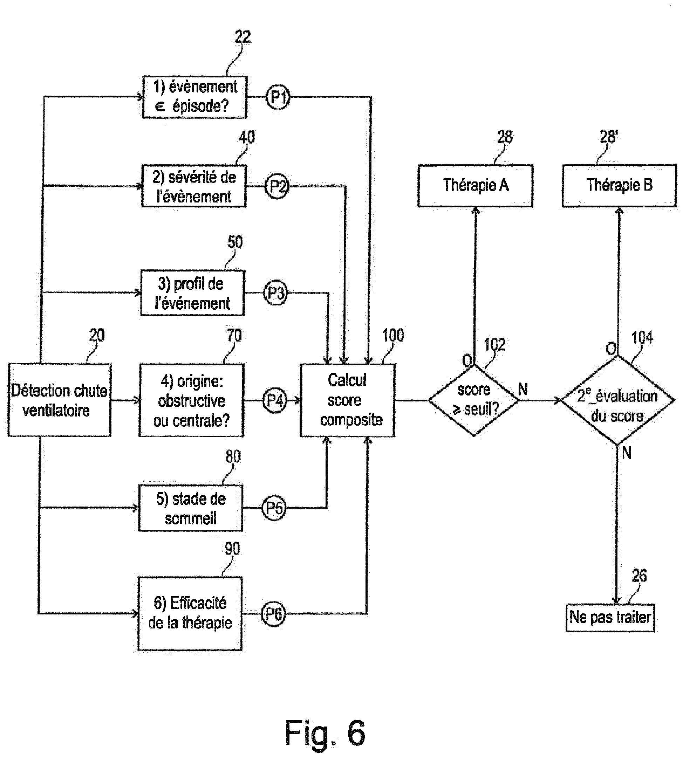

Comme illustré Figure 6, sur détection d'une chute ventilatoire (bloc 20) les différents critères sont évalués conjointement (blocs 22, 40, 50, 70, 80, 90).

À chaque critère est allouée une pondération P1 à P6, par exemple par attribution à chacun des critères d'un nombre de points, positifs ou négatifs constituant un score élémentaire.

Par exemple, les scores élémentaires P1 à P6 correspondant respectivement aux critères n°1 à n°6 exposés plus haut pourront prendre les valeurs suivantes :

- P1 = valeur du compteur NB_ÉPISODE (compteur décrit Figure 4, incrémenté à l'étape 32) ;
- P2 = +5 si SEUIL_SÉVÉRITÉ est franchi, 0 si le profil courant est inconnu, +3 si le profil courant correspond à un profil de référence connu ;
- P4 (facteur multiplicatif) = +1 pour une origine centrale prédominante ou par défaut une origine inconnue, -1 pour une origine prédominante obstructive ;
- P5 = -2 en sommeil paradoxal, -5 en veille, +1 en sommeil lent ;
- P6 = -3 si patient non répondeur au traitement, +2 si patient répondeur au traitement, 0 si réponse indéterminée.

Un score composite est calculé (bloc 100) par addition et/ou multiplication des points P1 à P6.

Dans l'exemple ci-dessus, le score composite sera calculé par addition des scores individuels P1 à P3 et P5 à P6, et multiplication par le score individuel P4 :

$$S = (P1+P2+P3+P5+P6) \times P4.$$

Le score est alors comparé à un seuil prédéterminé (test 102), par exemple un seuil égal à 5 avec les valeurs de l'exemple précédent, et si ce seuil est franchi une thérapie prédéterminée est appliquée ("Thérapie A", bloc 28).

Dans le cas contraire, et si plusieurs thérapies sont disponibles (notamment pour traiter différemment les hypopnées obstructives des hypopnées centrales), une seconde évaluation du score est effectuée (test 104) et selon le résultat une thérapie différente ("Thérapie B", bloc 28') est appliquée, ou alors il est décidé de n'appliquer aucune thérapie au patient (bloc 26). Avec les valeurs de l'exemple précédent, la Thérapie B est appliquée si le score est négatif mais sa valeur absolue dépasse le seuil, aucune thérapie n'étant appliquée dans le cas contraire.

Cette combinaison de critères permet de prendre en compte des cas de figure complexes et de déclencher sélectivement une thérapie dans des situations très spécifiques, par exemple :

- hypopnée de profil connu, survenant en sommeil lent ;
- deuxième hypopnée d'un épisode, pour un patient de type répondeur, en sommeil lent ;
- hypopnée de profil connu, en sommeil lent pour un patient répondeur ;
- quatrième hypopnée d'un épisode, de profil connu et survenant en sommeil paradoxal ;
- etc.

## Revendications

1. Un dispositif médical actif, comprenant :

   - des moyens (12) de mesure d'activité respiratoire, aptes à délivrer une valeur de débit représentative du débit ventilatoire instantané d'un patient ;
   - des moyens de détection (20) d'un événement de chute ventilatoire, aptes à détecter en temps réel une diminution de la valeur de débit en deçà d'un seuil prédéterminé ; et
   - des moyens de thérapie anti-hypopnée ;
   - des moyens discriminateurs, aptes à analyser en temps réel, pendant ledit événement de chute ventilatoire, au moins un paramètre de l'événement de chute ventilatoire courant et à déterminer si ce paramètre vérifie au moins deux critères prédéfinis, pour attribuer à l'événement un indicateur de suspicion a priori d'hypopnée ou bien de non-suspicion a priori d'hypopnée,

dans lequel :

- les au moins deux critères prédéfinis des moyens discriminateurs comprennent la valeur d'un compteur (NB_ÉPISODE) d'événements de chute ventilatoire successifs détectés dans l'intervalle d'une période prédéterminée (INTERVALLE_ÉPISODE) correspondant à une durée d'épisode d'hypopnées ;
- les moyens discriminateurs sont des moyens aptes à n'attribuer (30-34) un indicateur de suspicion d'hypopnée à l'événement courant que si la valeur du compteur dépasse une valeur minimale (SEUIL_ÉPISODE) prédéterminée ; et
- le dispositif comprend des moyens de remise à zéro (36) du compteur si le temps écoulé entre deux événements de chute ventilatoire successifs excède ladite période prédéterminée (INTERVALLE_ÉPISODE) correspondant à une durée d'épisode d'hypopnées ; et
- des moyens d'application sélective d'au moins une thérapie anti-hypopnée, aptes à déclencher (28) avant la fin dudit événement de chute ventilatoire, à partir du franchissement dudit seuil prédéterminé, une thérapie anti-hypopnée seulement en cas d'attribution à l'événement courant d'un indicateur de suspicion a priori d'hypopnée ;
- les au moins deux critères prédéfinis des moyens discriminateurs comprennent un profil de référence d'hypopnée conservé en mémoire ;
- le dispositif comprend des moyens aptes à déterminer un profil courant pour l'événement de chute ventilatoire courant et à évaluer une corrélation (50) entre le profil courant et le profil de référence ; et
- les moyens discriminateurs sont des moyens aptes à attribuer un indicateur de suspicion d'hypopnée à l'événement courant si la corrélation entre le profil courant et le profil de référence dépasse une valeur minimale de corrélation prédéterminée ; et
- le dispositif comprend en outre :
- des moyens aptes à avérer ou non (54), après la fin de l'évènement d'hypopnée suspectée (52), la présence d'une hypopnée consécutive à la détection de l'événement de chute ventilatoire ; et
- des moyens aptes à mettre à jour (56) ledit profil de référence d'hypopnée conservé en mémoire, en cas d'hypopnée avérée.

2. Le dispositif de la revendication 1, comprenant en outre :

- des moyens aptes à inhiber (26) toute thérapie anti-hypopnée en cas d'attribution à l'événement courant d'un indicateur de non-suspicion a priori d'hypopnée.

3. Le dispositif de la revendication 1 ou 2, comprenant en outre :

- des moyens (38) de mesure d'un signal physiologique fonction de l'activité respiratoire ;
- des moyens de détection (38) du franchissement d'un seuil limite (SEUIL_SÉVÉRITÉ) par le signal physiologique ; et
- des moyens pour forcer l'incrémentation (32) du compteur à toute détection d'un événement de chute ventilatoire.

4. Le dispositif de la revendication 1, 2 ou 3, dans lequel ladite période prédéterminée (INTERVALLE_ÉPISODE) et/ou ladite valeur minimale prédéterminée (SEUIL_ÉPISODE) sont des valeurs adaptatives variables, et dans lequel le dispositif comprend en outre :

- des moyens de calcul automatique de ladite période prédéterminée (INTERVALLE_ÉPISODE) et/ou de ladite valeur minimale prédéterminée (SEUIL_ÉPISODE).

5. Le dispositif de la revendication 1, comprenant en outre :

- des moyens (16) de mesure d'un signal physiologique fonction de l'activité respiratoire ;
- des moyens de détection (40) du franchissement d'un seuil limite (SEUIL_SÉVÉRITÉ) par un signal respiratoire ou par le signal physiologique ; et
- des moyens pour forcer le déclenchement (28) de la thérapie anti-hypopnée dès le franchissement dudit seuil limite.

6. Le dispositif de la revendication 3 ou 5, dans lequel le signal physiologique est un signal du groupe formé par : niveau de saturation sanguine en oxygène, taux de désaturation en oxygène sanguin, fréquence cardiaque, pression artérielle, onde de pouls, électroencéphalogramme, électromyogramme et les combinaisons des précédents.

7. Le dispositif de la revendication 1, dans lequel les moyens discriminateurs comprennent :

- des moyens aptes à analyser (22, 40, 50, 70, 80, 90) une pluralité de paramètres de l'événement de chute ventilatoire courant en fonction d'une pluralité de critères prédéfinis ;

- des moyens aptes à attribuer, pour l'événement de chute ventilatoire courant, un score spécifique respectif (P1-P6) à chacun desdits critères prédéfinis ;
- des moyens aptes à établir (100) un score composite global fonction d'une combinaison des scores spécifiques ; et
- des moyens pour attribuer (102) à l'événement courant un indicateur de suspicion a priori d'hypopnée ou bien de non-suspicion a priori d'hypopnée en fonction du résultat d'une comparaison du score global à un seuil de score prédéterminé.

8. Le dispositif de la revendication 7, dans lequel les moyens discriminateurs comprennent en outre :

- des moyens aptes à sélectionner (102, 104) en fonction de la valeur du score global une thérapie particulière parmi plusieurs thérapies possibles (28, 28').

9. Le dispositif de la revendication 1, dans lequel les au moins deux critères prédéfinis sont des critères du groupe formé par : historique des intervalles entre événements successifs de chute ventilatoire constitutifs d'épisodes d'hypopnées ; sévérité de l'événement de chute ventilatoire courant ; origine physiologique, obstructive ou centrale, de l'événement de chute ventilatoire ; stade de sommeil courant du patient ; et historique du degré d'efficacité des thérapies appliquées.

**Patentansprüche**

1. Aktive medizinische Vorrichtung, umfassend:

- Mittel (12) zur Messung der Atmungsaktivität, ausgestaltet zum Liefern eines Durchflusswerts, der für den momentanen Atemstrom eines Patienten repräsentativ ist;
- Mittel (20) zur Erfassung eines Atemsturzereignisses, ausgestaltet zum Erkennen einer Abnahme des Durchflusswertes unter einen vorbestimmten Schwellenwert in Echtzeit; und
- Mittel zur Anti-Hypopnoe-Therapie;
- Unterscheidungsmittel zum Analysieren mindestens eines Parameters des aktuellen Atemsturzereignisses in Echtzeit während des Atemsturzereignisses und zum Feststellen, ob dieser Parameter mindestens zwei vorbestimmte Kriterien erfüllt, um dem Ereignis einen Indikator für einen A-Priori-Verdacht auf Hypopnoe oder für einen A-Priori-Nichtverdacht auf Hypopnoe zuzuordnen;

wobei:

- die mindestens zwei vorbestimmten Kriterien der Unterscheidungsmittel den Wert eines Zählers (NB_EPISODE) für aufeinanderfolgende Atemsturzereignisse umfassen, die im Intervall eines vorbestimmten, einer Dauer einer Hypopnoe-Episode entsprechenden Zeitraums (INTERVALLE_ÉPISODE) festgestellt wurden;
- die Unterscheidungsmittel solche Mittel (30-34) sind, die ausgestaltet sind, dem aktuellen Ereignis nur dann einen Indikator für den Verdacht auf Hypopnoe zuzuordnen, wenn der Wert des Zählers einen vorbestimmten Mindestwert (SEUIL_ÉPISODE) überschreitet; und
- die Vorrichtung Mittel (36) umfasst zum Zurücksetzen des Zählers, wenn die Zeit, die zwischen zwei aufeinanderfolgenden Atemsturzereignissen verstrichen ist, den vorbestimmten, einer Dauer der Hypopnoe-Episode entsprechenden Zeitraum (INTERVALLE_ÉPISODE) überschreitet; und
- Mittel (28) zur selektiven Anwendung von mindestens einer Anti-Hypopnoe-Therapie, ausgestaltet zum Auslösen einer Anti-Hypopnoe-Therapie vor dem Ende des Atemsturzereignisses nach dem Überschreiten der vorbestimmten Schwelle nur im Falle der Zuordnung eines Indikators für einen A-Priori-Verdacht auf Hypopnoe zu dem aktuellen Ereignis;
- die mindestens zwei vorbestimmten Kriterien der Unterscheidungsmittel ein gespeichertes Referenzprofil der Hypopnoe umfassen;
- die Vorrichtung Mittel (50) umfasst, die ausgestaltet sind, ein aktuelles Profil für das aktuelle Atemsturzereignis zu bestimmen und eine Korrelation zwischen dem aktuellen Profil und dem Referenzprofil zu bewerten; und
- die Unterscheidungsmittel solche Mittel sind, die ausgestaltet sind, dem aktuellen Ereignis einen Indikator für den Verdacht auf Hypopnoe zuzuordnen, wenn die Korrelation zwischen dem aktuellen Profil und dem Referenzprofil einen vorbestimmten Mindestkorrelationswert überschreitet; und
- die Vorrichtung ferner umfasst:
- Mittel (54) zum Erkennen des Vorliegens einer Hypopnoe nach Feststellung des Atemsturzereignisses nach dem Ende des vermuteten Hypopnoe-Ereignisses (52) oder nicht; und
- Mittel (56) zum Aktualisieren (56) des gespeicherten Hypopnoe-Referenzprofils im Falle einer nachgewiesenen Hypopnoe.

2. Vorrichtung nach Anspruch 1, ferner umfassend:

- Mittel (26) zum Unterdrücken jeder Anti-Hypopnoe-Therapie im Falle der Zuordnung eines Indikators für den A-Priori-Nichtverdacht auf Hypopnoe zu dem aktuellen Ereignis.

**3.** Vorrichtung nach Anspruch 1 oder 2, ferner umfassend:

- Mittel (38) zur Messung eines von der Atmungsaktivität abhängigen physiologischen Signals;
- Mittel (38) zum Erfassen des Überschreitens eines Grenzwerts (SEUIL_SÉVÉRITÉ) durch das physiologische Signal; und
- Mittel (32) zum Erzwingen einer Erhöhung des Zählers bei jeder Erfassung eines Atemsturzereignisses.

**4.** Vorrichtung nach Anspruch 1, 2 oder 3, wobei der vorbestimmte Zeitraum (INTERVALLE_ÉPISODE) und/oder der vorbestimmte Mindestwert (SEUIL_ÉPISODE) variable adaptive Werte sind und wobei die Vorrichtung ferner umfasst:

- Mittel zur automatischen Berechnung des vorbestimmten Zeitraums (INTERVALLE_ÉPISODE) und/oder des vorbestimmten Mindestwerts (SEUIL_ÉPISODE).

**5.** Vorrichtung nach Anspruch 1, ferner umfassend:

- Mittel (16) zur Messung eines physiologischen Signals in Abhängigkeit von der Atmungsaktivität;
- Mittel (40) zum Nachweis des Überschreitens eines Grenzwerts (SEUIL_SÉVÉRITÉ) durch ein Atemsignal oder durch das physiologische Signal; und
- Mittel (28) zum Erzwingen des Auslösens der Anti-Hypopnoe-Therapie, sobald die genannte Grenzschwelle überschritten wird.

**6.** Vorrichtung nach Anspruch 3 oder 5, wobei das physiologische Signal ein Signal ist aus der Gruppe gebildet durch: Blutsauerstoffsättigungsgrad, Blutsauerstoffdesaturierungsrate, Herzfrequenz, Blutdruck, Pulswelle, Elektroenzephalogramm, Elektromyogramm und Kombinationen der vorhergehenden.

**7.** Vorrichtung nach Anspruch 1, wobei die Unterscheidungsmittel umfassen:

- Mittel (22, 40, 50, 70, 80, 90) zum Analysieren einer Vielzahl von Parametern des aktuellen Atemsturzereignisses nach einer Vielzahl vordefinierter Kriterien;
- Mittel zum Zuweisen einer jeweils spezifischen Punktzahl (P1-P6) zu jedem der vordefinierten Kriterien für das aktuelle Atemsturzereignis;
- Mittel (100) zum Ermitteln einer Gesamtpunktzahl als Funktion einer Kombination der spezifischen Punktzahlen; und
- Mittel (102) zum Zuweisen eines Indikators für einen A-Priori-Verdacht auf Hypopnoe oder einen A-Priori-Nichtverdacht auf Hypopnoe zu dem aktuellen Ereignis abhängig vom Ergebnis eines Vergleichs der Gesamtpunktzahl mit einer vorbestimmten Punktschwelle.

**8.** Vorrichtung nach Anspruch 7, wobei die Unterscheidungsmittel ferner umfassen:

- Mittel (102, 104) zum Auswählen einer bestimmte Therapie aus mehreren möglichen Therapien (28, 28') in Abhängigkeit des Werts der Gesamtpunktzahl.

**9.** Vorrichtung nach Anspruch 1, wobei die mindestens zwei vordefinierten Kriterien aus einer Gruppe von Kriterien gewählt sind, die gebildet wird durch: Geschichte der Intervalle zwischen aufeinanderfolgenden, Episoden von Hypopnoe bildenden Atemsturzereignissen; Schwere des aktuellen Atemsturzereignisses; physiologischer, obstruktiver oder zentraler Ursprung des Atemsturzereignisses; aktuelles Schlafstadium des Patienten; und Geschichte des Wirksamkeitsgrades angewandter Therapien.

## Claims

**1.** An active medical device, comprising:

- means (12) for measuring respiratory activity, capable of delivering a flow rate value representative of the instantaneous ventilatory flow rate of a patient;
- means (20) for detecting a ventilatory drop event, capable of detecting in real time a decrease in the flow rate value below a predetermined threshold; and
- anti-hypopnea therapy means;
- discriminating means capable of analysing in real time, during said ventilatory drop event, at least one parameter of the current ventilatory drop event, and determining whether this parameter meets at least two predefined criteria, in order to attribute to the event an indicator of a priori suspicion of hypopnea or else of a priori non-suspicion of hypopnea,

in which:

- the at least two predefined criteria of the discriminating means comprise the value of a counter (NB_EPISODE) of successive ventilatory drop events detected within the interval of a predetermined period (INTERVAL_EPISODE) corresponding to a hypopnea episode duration;
- the discriminating means are means capable of assigning (30-34) an indicator of hypopnea suspicion to the current event only if the value of the counter exceeds a predetermined minimum value (THRESHOLD_EPISODE); and

- the device comprises means for resetting (36) the counter if the elapsed time between two successive ventilatory drop events exceeds said predetermined period (INTERVAL_EPISODE) corresponding to a hypopnea episode duration, and means for selectively applying at least one anti-hypopnea therapy, capable of triggering (28) before the end of said ventilatory drop event, upon crossing of said predetermined threshold, an anti-hypopnea therapy only in case of assignment to the current event of an indicator of a priori suspicion of hypopnea,

- the at least two predefined criteria of the discriminating means comprise a hypopnea reference profile stored in memory;

- the device comprises means capable of determining a current profile for the current ventilatory drop event, and evaluating a correlation (50) between the current profile and the reference profile; and

- the discriminating means are means capable of assigning an indicator of suspected hypopnea to the current event if the correlation between the current profile and the reference profile exceeds a minimum value of predetermined correlation; and

- the device further comprises:

- means capable of ascertaining or not (54), after the end of the suspected hypopnea event (52), the presence of a hypopnea subsequent to the detection of the ventilatory drop event; and

- means capable of updating (56) said hypopnea reference profile stored in memory in case of ascertained hypopnea.

2. The device of claim 1, further comprising:

- means capable of inhibiting (26) any anti-hypopnea therapy in case of assignment to the current event of an indicator of a priori non-suspicion of hypopnea.

3. The device of claim 1 or 2, further comprising:

- means (38) for measuring a physiological signal dependent on the respiratory activity;

- means (38) for detecting the crossing of a threshold limit (THRESHOLD_SEVERITY) by the physiological signal; and

- means for forcing the incrementing (32) of the counter at any detection of a ventilatory drop event.

4. The device of claim 1, 2 or 3, wherein said predetermined period (INTERVAL_EPISODE) and/or said predetermined minimum value (THRESHOLD_EPISODE) are variable adaptive values, and wherein the device further comprises:

- means for automatically calculating said predetermined period (INTERVAL_EPISODE) and/or said predetermined minimum value (THRESHOLD_EPISODE).

5. The device of claim 1, further comprising:

- means (16) for measuring a physiological signal dependent on the respiratory activity;

- means (40) for detecting the crossing of a threshold limit (THRESHOLD_SEVERITY) by a respiratory signal or by the physiological signal; and

- means for forcing the triggering (28) of the anti-hypopnea therapy as soon as said threshold limit is crossed.

6. The device of claim 3 or 5, wherein the physiological signal is a signal from the group consisting of: blood oxygen saturation level, blood oxygen desaturation level, heart rate, blood pressure, pulse wave, electroencephalogram, electromyogram, and combinations thereof.

7. The device of claim 1, wherein the discriminating means comprise:

- means capable of analysing (22, 40, 50, 70, 80, 90) a plurality of parameters of the current ventilatory drop event according to a plurality of predefined criteria;

- means capable of assigning, for the current ventilatory drop event, a respective specific score (P1-P6) to each of said predefined criteria;

- means capable of establishing (100) an overall composite score dependent on a combination of the specific scores; and

- means for assigning (102) to the current event an indicator of a priori suspicion of hypopnea or else of a priori non-suspicion of hypopnea according to the result of a comparison of the overall score with a predetermined score threshold.

8. The device of claim 7, wherein the discriminating means further comprise:

- means capable of selecting (102, 104) according to the value of the overall score, a particular therapy from a plurality of possible therapies (28, 28').

9. The device of claim 1, wherein the at least one predefined criterion is a criterion from the group consisting of: history of intervals between successive ventilatory drop events constituting hypopnea episodes; severity of the current ventilatory drop event; physiological origin, obstructive or central, of the ventilatory drop event; current sleep stage of the patient; and history of the degree of effectiveness of the applied therapies.

# Fig. 1

Fig. 2a

Fig. 2b

_40_

Signal physiologique <
SEUIL_SÉVÉRITÉ

(déclenchement immédiat) ────────────────►

_28_

Thérapie A

_20_

Détection chute
ventilatoire

_22_

analyse

_24_

chute ventilatoire
∈ épisode?

O

N

_26_

Ne pas
traiter

# Fig. 3

_38_

Détection désaturation O₂

_20_

Détection
chute ventilatoire

_26_

Ne pas traiter

_30_

Intervalle entre chutes
ventilatoires ≤
INTERVALLE_ÉPISODE
?

N

_36_

O→NB_ÉPISODE

O

_32_

+1→NB_ÉPISODE

_34_

NB_ÉPISODE
≥ SEUIL_ÉPISODE
?

N

O

_28_

Thérapie A

# Fig. 4

40

Signal physiologique <
SEUIL_SÉVÉRITÉ

(déclenchement immédiat)

Détection chute
ventilatoire

20

50

corrélation
profil courant vs. profil
de référence?

O

28

Thérapie A

N

Ne pas
traiter

26

Attente reprise
ventilatoire

52

58

Hypopnée
avérée?

N

Mémoriser caractéristiques de faux positif

60

caractéristiques
déjà connues

O

54

O

Mémoriser
le profil

56

Nouveau profil
de faux positif

62

# Fig. 5

**Fig. 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1336422 A1
- US 200330153956 A1
- US 7371220 B1
- US 7942824 B1
- US 2010307500 A1
- WO 2007141345 A1
- FR 2908624 A1

**Littérature non-brevet citée dans la description**

- *AASM Manual for The Scoring of Sleep and Associated Events,* 2007
- Journal of Clinical Sleep Medicine. l'American Academy of Sleep Medicine, 2012, vol. 8
- **RENDERATH et al.** *Sleep,* 2013, vol. 36 (3), 363-368